# EUROPEAN PATENT APPLICATION

(11) **EP 4 420 774 A1**
(43) Date of publication of application: **28.08.2024**
(21) Application number: 22886122.5
(22) Date of filing: 28.10.2022
(51) Int. Cl.: B01J 23/22

(54) **CATALYST FOR GAS-PHASE OXIDATION OF 1,2,4,5-TETRAALKYLBENZENE, PREPARATION METHOD FOR AND APPLICATION OF CATALYST, AND PREPARATION METHOD FOR BENZENE-1,2,4,5-TETRACARBOXYLIC DIANHYDRIDE**

(30) Priority: 01.11.2021 CN 202111282786
(71) Applicant: China Petroleum & Chemical Corporation, Beijing 100728 (CN); Sinopec (Beijing) Research Institute of Chemical Industry Co., Ltd., Beijing 100013 (CN)
(72) Inventor: AN, Xin, Beijing 100013 (CN); YUAN, Bin, Beijing 100013 (CN); LIU, Yufen, Beijing 100013 (CN); SHI, Huimin, Beijing 100013 (CN); ZHANG, Dongshun, Beijing 100013 (CN); ZHANG, Zuofeng, Beijing 100013 (CN)
(74) Representative: Hoffmann Eitle
(86) International application number: PCT/CN2022/128240
(87) International publication number: WO 2023/072247

(57) **Abstract**

Disclosed in the present invention are a catalyst for gas-phase oxidation of 1,2,4,5-tetraalkylbenzene to prepare benzene-1,2,4,5-tetracarboxylic dianhydride, a preparation method for and an application of the catalyst, and a preparation method for benzene-1,2,4,5-tetracarboxylic dianhydride. The catalyst according to the present invention comprises a carrier and a catalytically active component coating attached to the carrier; the catalytically active component coating comprises a first coating and a second coating; the first coating is close to the surface of the carrier, and the second coating is distant from the surface of the carrier; in the first coating, the mass ratio of a titanium element denoted by Ti to a vanadium element denoted by V is Ti/V¹; in the second coating, the mass ratio of the titanium element denoted by Ti to the vanadium element denoted by V is Ti/V², wherein Ti/V²=Ti/V¹+ΔTi/V, and ΔTi/V is within a range of 3 to 9. The catalyst according to the present invention exhibits improved catalytic activity, and can effectively increase the yield of benzene-1,2,4,5-tetracarboxylic dianhydride.

## Description

### Cross-Reference to Related Applications

This application claims the benefit of Chinese patent application 202111282786.7, filed on November 1, 2021, the contents of which are incorporated herein by reference.

### Field of the Invention

The present invention relates to a catalyst, in particular to a catalyst for gas-phase oxidation of 1,2,4,5-tetraalkylbenzene to prepare benzene-1,2,4,5-tetracarboxylic dianhydride, and the present invention also relates to a preparation method for and an application of the catalyst, and the present invention further relates to a preparation method for benzene-1,2,4,5-tetracarboxylic dianhydride.

### Background of the Invention

Pyromellitic dianhydride (benzene-1,2,4,5-tetracarboxylic dianhydride, hereinafter referred to as a homoanhydride) is a very important chemical feedstock. Homoanhydrides and their derivatives have very important and broad applications, in particular the homoanhydrides can be used as one of main monomers in the production of polyimide. Polyimide is currently a new engineering material having the widest use temperature and excellent dimensional stability at high temperature, radiation resistance, mechanical properties, electrical properties, and corrosion resistance in organic polymer materials due to its comprehensive properties at high temperature.

Early, preparation of the homoanhydrides from durene as a raw material mainly adopts a liquid-phase oxidation method, wherein the raw material is subjected to liquid-phase oxidation to form an acid, and the acid is then subjected to dehydration to prepare an anhydride. At present, a commonly used process is a gas-phase oxidation method: pyromellitic dianhydride is prepared from 1,2,4,5-tetraalkylbenzene as a raw material in one step by air oxidation. The gas-phase oxidation method has the characteristics that the process is simple, a step of acid dehydration to the anhydride is omitted, air is used as an oxidant, there is no need of a catalyst separation step in the liquid-phase oxidation process, continuous production can be achieved, and automatic operation is easy to achieve.

Since the oxidation of 1,2,4,5-tetraalkylbenzene to prepare the homoanhydride is a highly exothermic reaction process in selective oxidation, the theoretical yield of the homoanhydride is high but the actual yield is low.

Regulating the elemental composition of a catalyst is one of the technical means mainly adopted in the prior art to increase the yield of the homoanhydride. CN107866241A discloses a catalyst for oxidation of durene to prepare a homoanhydride, the catalyst employs an inert carrier, and an active component comprises at least one of a vanadium element, a titanium element, a group VA element and an alkali metal element; and CN107866257A discloses a catalyst for preparation of a homoanhydride from durene, the catalyst uses α-Al₂O₃, silicon carbide, a porcelain ring or a mixture thereof as a carrier, and an active component comprises at least one of a vanadium element, an iron-based element, a group IIB element and an alkali metal element.

Staged loading of the catalyst is another technical means of increasing the yield of the homoanhydride in the prior art. CN1116197A discloses a method for producing benzene-1,2,4,5-tetracarboxylic dianhydride, and in the method, a catalyst is loaded in a reactor in stages to form a multi-stage catalyst, wherein a first catalyst is loaded on a product gas outlet side of the reactor, the first catalyst comprises vanadium (a) and at least one metal (b) selected from molybdenum and tungsten, and an atomic ratio of the metal (b) to vanadium (a) is 0.01-2; and at least one of other types of catalysts selected from a second catalyst and a third catalyst is loaded on a raw material mixed gas inlet side of the reactor, wherein the second catalyst comprises vanadium (a) and at least one metal (b) selected from molybdenum and tungsten, and an atomic ratio of the metal (b) to vanadium (a) is less than that of the first catalyst, the third catalyst comprises vanadium (a) and an alkali metal (c), and an atomic ratio of the alkali metal (c) to vanadium (a) is 0.2-2.5.

Although the regulation of the elemental composition of the catalyst and the staged loading of the catalyst have achieved certain effects in increasing the yield of the homoanhydride, there is still a large gap between the actual yield and the theoretical yield of the homoanhydride, and how to further increase the actual yield of the homoanhydride is still an urgent technical problem to be solved.

### Summary of the Invention

For most of the existing vanadium-based catalysts for gas-phase oxidation of 1,2,4,5-tetraalkylbenzene to prepare a homoanhydride, a coating comprising a catalytic active component is formed on a surface of a carrier, the inventors of the present invention have intensively studied the problem of low product yield during gas-phase oxidation of durene to prepare the homoanhydride in the prior art, and found that when the coating comprising the catalytically active component is formed on the surface of the carrier of the vanadium-based catalyst, a titanium/vanadium mass ratio in a radial direction of the coating is controlled so that the titanium/vanadium mass ratio in the coating close to the carrier is lower than the titanium/vanadium mass ratio in the coating distant from the carrier, and a difference between the two titanium/vanadium mass ratios is controlled to be within a certain range, thus effectively improving the yield of the homoanhydride. The present invention has been accomplished based on the above findings.

According to a first aspect of the present invention, the present invention provides a catalyst for gas-phase oxidation of 1,2,4,5-tetraalkylbenzene, comprising a carrier and a catalytically active component coating supported on the carrier, wherein the catalytically active component coating comprises a first coating and a second coating, the first coating is close to the surface of the carrier, the second coating is distant from the surface of the carrier, the first coating and the second coating each independently comprise vanadium element and titanium element, a mass ratio of the titanium element in terms of Ti to the vanadium element in terms of V in the first coating is Ti/V¹, a mass ratio of the titanium element in terms of Ti to the vanadium element in terms of V in the second coating is Ti/V², and Ti/V²=Ti/V¹+ΔTi/V, ΔTi/V being in range of 3 to 9.

According to a second aspect of the present invention, the present invention provides a preparation method for a catalyst for gas-phase oxidation of 1,2,4,5-tetraalkylbenzene, comprising the steps of:
(1) coating surface of a carrier with a first slurry to form a first coating, the first slurry comprising a first dispersion medium, a first vanadium source and a first titanium source; and
(2) coating the surface of the carrier where the first coating is formed with a second slurry to form a second coating, the second slurry comprising a second dispersion medium, a second vanadium source, a second titanium source, and a pore-expanding agent; wherein
a mass of the vanadium source introduced in the first coating is C_{V}¹², a mass of the titanium source introduced in the first coating is C_{Ti}¹², a mass of the vanadium source introduced in the second coating is C_{V}²², a mass of the titanium source introduced in the second coating is C_{Ti}²² , Ti/V¹²= C_{Ti}¹²/C_{V}¹², Ti/V²²=C_{Ti}²²/C_{V}²², and Ti/V²²=Ti/V¹²+ΔTi/V², ΔTi/V² being in range of 3 to 9, the vanadium source being in terms of V, and the titanium source being in terms of Ti.

According to a third aspect of the present invention, the present invention provides a catalyst prepared by the method in the second aspect of the present invention.

According to a fourth aspect of the present invention, the present invention provides application of the catalyst according to the first aspect or the third aspect of the present invention as a catalyst for a reaction for oxidation of 1,2,4,5-tetraalkylbenzene to prepare benzene-1,2,4,5-tetracarboxylic dianhydride.

According to a fifth aspect of the present invention, the present invention provides a preparation method for benzene-1,2,4,5-tetracarboxylic dianhydride, comprising contacting 1,2,4,5-tetraalkylbenzene and an oxygen-containing gas with the catalyst according to the first aspect or the third aspect of the present invention to obtain a product gas stream comprising benzene-1,2,4,5-tetracarboxylic dianhydride.

The catalyst according to the present invention exhibits improved catalytic activity as the catalyst for the reaction for the oxidation of 1,2,4,5-tetraalkylbenzene to prepare benzene-1,2,4,5-tetracarboxylic dianhydride, and can effectively increase the yield of benzene-1,2,4,5-tetracarboxylic dianhydride.

### Detailed Description of the Embodiments

The endpoints and any values of the ranges disclosed herein are not limited to the precise range or value, and these ranges or values should be understood as including values close to these ranges or values. For numerical ranges, the endpoint values of each range, the endpoint values of each range and individual point values, and individual point values may be combined with each other to obtain one or more new numerical ranges, and these numerical ranges should be considered to be specifically disclosed herein.

In the present invention, unless otherwise specified, "first" and "second" appearing before a material name or a step do not indicate a sequential order nor a limitation to each material or step, but are only used to distinguish the material name or the step. For example, "first" and "second" in a "first coating" and a "second coating" are merely used to distinguish the two coatings. In the present invention, the term "optional" means that the term following "optional" is not essential and can be understood as comprising or not comprising, including or excluding the term.

In the present invention, 1,2,4,5-tetraalkylbenzene may be one or two or more selected from compounds represented by a formula I: 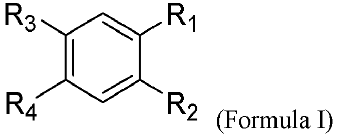 in the formula I, R₁, R₂, R₃ and R₄ are the same or different and are each independently C₁-C₅ alkyl. In the present invention, the C₁-C₅ alkyl includes C₁-C₅ linear alkyl and C₃-C₅ branched alkyl, and specific examples of the C₁-C₅ alkyl may include, but are not limited to, methyl, ethyl, n-propyl, isopropyl, n-butyl, sec-butyl, isobutyl, tert-butyl, n-pentyl, isopentyl, tert-pentyl and neopentyl.

Preferably, 1,2,4,5-tetraalkylbenzene is 1,2,4,5-tetramethylbenzene (i.e., durene).

According to a first aspect of the present invention, the present invention provides a catalyst for gas-phase oxidation of 1,2,4,5-tetraalkylbenzene. According to the catalyst of the present invention, the catalyst comprises a carrier and a catalytically active component coating supported on the carrier, the catalytically active component coating comprises a first coating and a second coating, the first coating is close to the surface of the carrier, and the second coating is distant from the surface of the carrier.

According to the catalyst of the present invention, the first coating and the second coating each independently comprise vanadium element and titanium element. According to the catalyst of the present invention, a mass ratio of the titanium element to the vanadium element (i.e., a mass ratio of Ti/V) in the first coating and the second coating is different, wherein a mass ratio of Ti/V² in the second coating is higher than a mass ratio of Ti/V¹ in the first coating. The inventors of the present invention found during the research process that in the catalyst, the mass ratio of Ti/V¹ in the first coating which is an inner coating close to the surface of the carrier is lower than the mass ratio of Ti/V² in the second coating which is an outer coating distant from the surface of the carrier, and a difference between the two mass ratios is controlled to be within a specific range, so that the activity of the catalyst can be effectively improved and a higher product yield can be obtained. According to the catalyst of the present invention, a mass ratio of the titanium element in terms of Ti to the vanadium element in terms of V in the first coating is Ti/V¹, a mass ratio of the titanium element in terms of Ti to the vanadium element in terms of V in the second coating is Ti/V², and Ti/V²=Ti/V¹+ΔTi/V, ΔTi/V being in range of 3 to 9. Specifically, ΔTi/V can be 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, or 9. Preferably, ΔTi/V is in range of 3.5 to 8.5.

According to the catalyst of the present invention, the mass ratio Ti/V¹ of the titanium element in terms of Ti to the vanadium element in terms of V in the first coating may be 7-8.5:1, and may be, for example, 7:1, 7.1:1, 7.2:1, 7.3:1, 7.4:1, 7.5:1, 7.6:1, 7.7:1, 7.8:1, 7.9:1, 8:1, 8.1:1, 8.2:1, 8.3:1, 8.4:1 or 8.5:1.

According to the catalyst of the present invention, the mass ratio Ti/V² of the titanium element in terms of Ti to the vanadium element in terms of V in the second coating may be 12-15.5: 1, and may be, for example, 12:1, 12.1:1, 12.2:1, 12.3:1, 12.4:1, 12.5:1, 12.6:1, 12.7:1, 12.8:1, 12.9:1, 13:1, 13.1:1, 13.2:1, 13.3:1, 13.4:1, 13.5:1, 13.6:1, 13.7:1, 13.8:1, 13.9:1, 14:1, 14.1:1, 14.2:1, 14.3:1, 14.4:1, 14.5:1, 14.6:1, 14.7:1, 14.8:1, 14.9:1, 15:1, 15.1:1, 15.2:1, 15.3:1, 15.4:1 or 15.5:1.

According to the catalyst of the present invention, the mass percentage content C_{Ti}¹ of the titanium element in the first coating in terms of Ti may be in the range of 40-60%, and may be, for example, 40%, 41%, 42%, 43%, 44%, 45%, 46%, 47%, 48%, 49%, 50%, 51%, 52%, 53%, 54%, 55%, 56%, 57%, 58%, 59% or 60%. Preferably, the mass percentage content C_{Ti}¹ of the titanium element in the first coating in terms of Ti is in the range of 45-55%.

According to the catalyst of the present invention, the mass percentage content C_{Ti}² of the titanium element in the second coating in terms of Ti may be in the range of 45-65%, and may be, for example, 45%, 46%, 47%, 48%, 49%, 50%, 51%, 52%, 53%, 54%, 55%, 56%, 57%, 58%, 59%, 60%, 61%, 62%, 63%, 64% or 65%. Preferably, the mass percentage content C_{Ti}² of the titanium element in the second coating in terms of Ti is in the range of 50-60%.

In the present invention, the mass ratio of the titanium element to the vanadium element (i.e., the mass ratio of Ti/V) in the first coating and the second coating is determined by a scanning electron microscope-energy dispersive spectrometer, and a specific test method is as follows: after catalyst particles are cooled with liquid nitrogen, the catalyst particles are cut from one side having no coating in a longitudinal direction to obtain catalyst semi-particles with an exposed cross section, the catalyst semi-particles are fixed to a conductive tape, and gold, platinum or carbon is sprayed onto the surfaces to increase their conductivity to obtain a test sample; and the test sample is placed in a sample stage of the scanning electron microscope, the cross section of the catalyst semi-particles is observed by using the scanning electron microscope, and the mass ratio of Ti/V of the coating at different thicknesses (also referred to as the "longitudinal direction") is measured by using the energy dispersive spectrometer, wherein a method for determining the mass ratio of Ti/V at the same coating thickness is as follows: three immediately adjacent points are randomly selected along a horizontal direction of the coating (i.e., a direction perpendicular or substantially perpendicular to a thickness direction of the coating), and magnified by the same factor (typically, the magnification factor is 500-1000X), the mass ratio of Ti/V at the point is measured by using the energy dispersive spectrometer, and values measured at the three points are averaged as the mass ratio of Ti/V in the horizontal direction. In the present invention, the first coating and the second coating are distinguished by a difference of the mass ratio of Ti/V between two adjacent points in the thickness direction of the coating, if the difference of the mass ratio of Ti/V between two adjacent points is within the range of ΔTi/V defined in the present invention, then the two points belong to the first coating and the second coating; and if the difference of the mass ratio of Ti/V between two adjacent points is less than the range ΔTi/V defined in the present invention (including two endpoint values), then the two points belong to the same coating.

According to the catalyst of the present invention, the catalytically active component coating may also comprise trace regulating elements. In a preferred embodiment, the catalytically active component coating further comprises at least one element selected from the group consisting of group VA non-metallic element, alkali metal element and assistant metal element, and the assistant metal element is at least one selected from the group consisting of rare earth element, group VIB element, group VIII element, group IIIA metal element, group VA metal element, and group IVB element other than titanium element.

According to the catalyst of the present invention, the group VA non-metallic element is preferably phosphorus (P). In the first coating, the mass percentage content of the group VA non-metallic element in terms of an element may be 0.1-0.5%, preferably 0.3-0.4%. According to the catalyst of the present invention, the second coating may or may not comprise the group VA non-metallic element. Preferably, the second coating does not comprise the group VA non-metallic element.

According to the catalyst of the present invention, the alkali metal element is preferably cesium (Cs). In the first coating, the mass percentage content of the alkali metal element in terms of an element may be 0.1-0.3%, preferably 0.15-0.2%. In the second coating, the mass percentage content of the alkali metal element in terms of an element may be 0.1-0.7%, preferably 0.3-0.5%.

According to the catalyst of the present invention, specific examples of the assistant metal element may include, but are not limited to, at least one selected from the group consisting of rubidium (Rb), cerium (Ce), niobium (Nb), chromium (Cr), tungsten (W), silver (Ag), cobalt (Co), gallium (Ga), indium (In), antimony (Sb), bismuth (Bi), zirconium (Zr), and erbium (Er). In a preferred embodiment, the assistant metal element is at least one selected from the group consisting of niobium, zirconium, and antimony.

According to the catalyst of the present invention, the mass percentage content of the assistant metal element in the first coating in terms of an oxide may be 1-6%, preferably 1.5-5%, more preferably 2-4.5%, further preferably 3-4%. In a preferred embodiment, the assistant metal elements in the first coating are niobium, zirconium and antimony. In this preferred embodiment, in the first coating, the mass percentage content of a niobium element-containing compound in terms of Nb₂O₅ may be 0.15-0.2%, the mass percentage content of a zirconium element-containing compound in terms of ZrO₂ may be 0.4-0.45%, and the mass percentage content of an antimony element-containing compound in terms of Sb₂O₃ may be 2.8-3.2%.

In the second coating, the mass percentage content of the assistant metal element in terms of an oxide may be 1-6%, preferably 1.2-5%, more preferably 1.5-3%. In a preferred embodiment, the assistant metal elements in the second coating are niobium and antimony. In this preferred embodiment, in the second coating, the mass percentage content of a niobium element-containing compound in terms of Nb₂O₅ may be 0.12-0.18%, and the mass percentage content of an antimony element-containing compound in terms of Sb₂O₃ may be 1.5-2%.

In the present invention, the contents of the titanium element, the group VA non-metallic element, the alkali metal element and the assistant metal element in the coating are calculated by the feeding amount based on the total amount (in terms of oxides) of raw materials forming the coating, and the raw materials do not include the dispersion medium.

According to the catalyst of the present invention, the first coating is at a shorter distance from the carrier than the second coating, i.e., the first coating is closer to the surface of the carrier than the second coating. In a preferred embodiment, the second coating is attached to a surface of the first coating. In a more preferred embodiment, the second coating is attached to the surface of the first coating, and the first coating is attached to the surface of the carrier. In this more preferred embodiment, the second coating is exposed to the environment as an outer coating and the first coating connects the carrier with the second coating as an inner coating.

According to the catalyst of the present invention, a weight ratio of the first coating to the second coating to the carrier may be 5.5-6.8:1.9-3.5:100, preferably 5.8-6.7:1.9-2.9:100.

According to the catalyst of the present invention, the catalyst has a total pore volume of 0.03-0.08 mL/g.

In the present invention, the total pore volume of the catalyst is determined by using a BET specific surface method.

According to the catalyst of the present invention, the carrier is used for supporting the catalytically active component coating, and the carrier is an inert inorganic carrier and may be at least one selected from the group consisting of alumina, talc, silicon carbide, aluminum silicate, quartz and ceramic. According to the catalyst of the present invention, a shape of the catalyst may be cylindrical, spherical, annular or granular. Preferably, the shape of the catalyst is annular. In a preferred embodiment, the catalyst is annular, and an outer diameter of the catalyst (i.e., a diameter of an outer ring) may be 3-13 mm, and may be, for example, 3 mm, 4 mm, 5 mm, 6 mm, 7 mm, 8 mm, 9 mm, 10 mm, 11 mm, 12 mm or 13 mm. Preferably, the catalyst has an outer diameter of 5-9 mm. In this preferred embodiment, the catalyst has a wall thickness (i.e., a difference between the diameter of the outer ring and a diameter of an inner ring/2) of 0.1-10 mm, preferably 0.1-5 mm, more preferably 1-3 mm. In this preferred embodiment, a height of the catalyst may be 2-12 mm, and may be, for example, 2 mm, 3 mm, 4 mm, 5 mm, 6 mm, 7 mm, 8 mm, 9 mm, 10 mm, 11 mm or 12 mm, preferably 3-8 mm.

According to a second aspect of the present invention, the present invention provides a preparation method for a catalyst for gas-phase oxidation of 1,2,4,5-tetraalkylbenzene, comprising the steps of:
(1) coating a surface of a carrier with a first slurry to form a first coating, the first slurry comprising a first dispersion medium, a first vanadium source and a first titanium source; and
(2) coating the surface of the carrier where the first coating is formed with a second slurry to form a second coating, the second slurry comprising a second dispersion medium, a second vanadium source, a second titanium source, and a pore-expanding agent.

According to the method of the present invention, a mass of the vanadium source introduced in the first coating is C_{V}¹², a mass of the titanium source introduced in the first coating is C_{Ti}¹², a mass of the vanadium source introduced in the second coating is Cᵥ²², a mass of the titanium source introduced in the second coating is C_{Ti}²², Ti/V¹²=C_{Ti}¹²/C_{V}¹², Ti/V²²=C_{Ti}²² /C_{V}²², and Ti/V²²=Ti/V¹²+ΔTi/V², ΔTi/V² being in the range of 3 to 9, the vanadium source being in terms of V, and the titanium source being in terms of Ti. Specifically, ΔTi/V² can be 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, or 9. Preferably, ΔTi/V² is in the range of 3.5 to 8.5.

According to the preparation method for the catalyst of the present invention, the mass percentage content C_{Ti}¹² of the titanium source introduced in the first coating in terms of Ti may be in the range of 40-60%, and may be, for example, 40%, 41%, 42%, 43%, 44%, 45%, 46%, 47%, 48%, 49%, 50%, 51%, 52%, 53%, 54%, 55%, 56%, 57%, 58%, 59% or 60%. Preferably, the mass percentage content C_{Ti}¹² of the titanium element introduced in the first coating in terms of Ti is in the range of 45-55%. According to the preparation method for the catalyst of the present invention, the mass percentage content C_{Ti}²² of the titanium source introduced in the second coating in terms of Ti may be in the range of 45-65%, and may be, for example, 45%, 46%, 47%, 48%, 49%, 50%, 51%, 52%, 53%, 54%, 55%, 56%, 57%, 58%, 59%, 60%, 61%, 62%, 63%, 64% or 65%. Preferably, the mass percentage content C_{Ti}²² of the titanium element introduced in the second coating in terms of Ti is in the range of 50-60%. According to the preparation method for the catalyst of the present invention, the first vanadium source and the second vanadium source may be the same or different. The first vanadium source and the second vanadium source may each independently be at least one selected from the group consisting of ammonium metavanadate, vanadium pentoxide, and sodium vanadate. Preferably, the first vanadium source and the second vanadium source are ammonium metavanadate.

According to the preparation method for the catalyst of the present invention, the first titanium source and the second titanium source may be the same or different. The first titanium source and the second titanium source may each independently be titanium dioxide and/or a precursor of titanium dioxide. In a preferred embodiment, the first titanium source and the second titanium source are each independently at least one selected from the group consisting of titanium dioxide and metatitanic acid. The titanium dioxide is preferably anatase TiO₂. The anatase TiO₂ may have a specific surface area of 10-30 m²/g.

According to the preparation method for the catalyst of the present invention, the mass ratio Ti/V¹² of the titanium element introduced in the first coating in terms of Ti to the vanadium element introduced in the first coating in terms of V may be 7-8.5:1, and may be, for example, 7:1, 7.1:1, 7.2:1, 7.3:1, 7.4:1, 7.5:1, 7.6:1, 7.7:1, 7.8:1, 7.9:1, 8:1, 8.1:1, 8.2:1, 8.3:1, 8.4:1 or 8.5:1.

According to the preparation method for the catalyst of the present invention, the mass ratio Ti/V²² of the titanium element introduced in the second coating in terms of Ti to the vanadium element introduced in the second coating in terms of V may be 12-15.5:1, and may be, for example, 12:1, 12.1:1, 12.2:1, 12.3:1, 12.4:1, 12.5:1, 12.6:1, 12.7:1, 12.8:1, 12.9:1, 13:1, 13.1:1, 13.2:1, 13.3:1, 13.4:1, 13.5:1, 13.6:1, 13.7:1, 13.8:1, 13.9:1, 14:1, 14.1:1, 14.2:1, 14.3:1, 14.4:1, 14.5:1, 14.6:1, 14.7:1, 14.8:1, 14.9:1, 15:1, 15.1:1, 15.2:1, 15.3:1, 15.4:1 or 15.5:1.

According to the preparation method for the catalyst of the present invention, the first slurry and the second slurry may each independently comprise at least one selected from the group consisting of a compound containing group VA non-metallic element, a compound containing alkali metal element and a compound containing assistant metal element, such that the first coating and the second coating each independently contain at least one selected from the group consisting of the compound containing group VA non-metallic element, the compound containing alkali metal element and the compound containing assistant metal element. The assistant metal element is at least one selected from the group consisting of rare earth element, group VIB element, group VIII element, group IIIA metal element, group VA metal element, and group IVB element other than titanium element. In one embodiment, the first slurry comprises the compound containing group VA non-metallic element, the compound containing alkali metal element and the compound containing assistant metal element. The second slurry comprises the compound containing alkali metal element, the compound containing assistant metal element, and optionally the compound containing group VA non-metallic element.

According to the preparation method for the catalyst of the present invention, the group VA non-metallic element is preferably phosphorus (P). Preferably, the compound containing group VA non-metallic element is at least one selected from the group consisting of ammonium dihydrogen phosphate, triammonium phosphate, and phosphorus pentoxide. The mass percentage content of the compound containing group VA non-metallic element introduced in the first coating in terms of an element may be 0.1-0.5%, preferably 0.3-0.4%. According to the preparation method for the catalyst of the present invention, the second coating may or may not comprise the compound containing group VA non-metallic element. The second slurry may or may not comprise the group VA non-metallic element. Preferably, the second slurry does not comprise the group VA non-metallic element. According to the preparation method for the catalyst of the present invention, the alkali metal element is preferably cesium (Cs). Preferably, the compound containing alkali metal element is at least one selected from the group consisting of MNO₃, M₂SO₄, MCl and M₂CO₃, M being the alkali metal element. The mass percentage content of the compound containing alkali metal element introduced in the first coating in terms of an element may be 0.1-0.3%, more preferably 0.15-0.2%. The mass percentage content of the compound containing alkali metal element introduced in the second coating in terms of an element may be 0.1-0.7%, more preferably 0.3-0.5%.

According to the preparation method for the catalyst of the present invention, specific examples of the assistant metal element may include, but are not limited to, at least one selected from the group consisting of rubidium (Rb), cerium (Ce), niobium (Nb), chromium (Cr), tungsten (W), silver (Ag), cobalt (Co), gallium (Ga), indium (In), antimony (Sb), bismuth (Bi), zirconium (Zr), and erbium (Er). In a preferred embodiment, the assistant metal element is at least one selected from the group consisting of niobium, zirconium, and antimony. Preferably, the compound containing assistant metal element is at least one selected from the group consisting of an oxide of an assistant metal and a water-soluble salt containing the assistant metal. The water-soluble metal salt refers to a substance having a solubility in water of 1 g/100 g water or more, and may be at least one selected from the group consisting of nitrate, carbonate, sulfate, oxalate and chloride.

According to the preparation method for the catalyst of the present invention, the mass percentage content of the compound containing assistant metal element introduced in the first coating in terms of an oxide may be 1-6%, preferably 1.5-5%, more preferably 2-4.5%, further preferably 3-4%. The mass percentage content of the compound containing assistant metal element introduced in the second coating in terms of an oxide may be 1-6%, preferably 1.2-5%, more preferably 1.5-3%.

According to the preparation method for the catalyst of the present invention, in a preferred embodiment, the assistant metal elements in the first slurry are niobium, zirconium and antimony. In this preferred embodiment, the mass percentage content of a niobium element-containing compound introduced in the first coating in terms of Nb₂O₅ may be 0.15-0.2%, the mass percentage content of a zirconium element-containing compound introduced in the first coating in terms of ZrO₂ may be 0.4-0.45%, and the mass percentage content of an antimony element-containing compound introduced in the first coating in terms of Sb₂O₃ may be 2.8-3.2%.

According to the preparation method for the catalyst of the present invention, in a preferred embodiment, the assistant metal elements in the second coating are niobium and antimony. In this preferred embodiment, the mass percentage content of a niobium element-containing compound introduced in the second coating in terms of Nb₂O₅ may be 0.12-0.18%, and the mass percentage content of an antimony element-containing compound introduced in the second coating in terms of Sb₂O₃ may be 1.5-2%.

In the preparation method for the catalyst of the present invention, the content of each element introduced in the first coating and the second coating is calculated by the feeding amount on the basis of the total amount (in terms of oxides) of raw materials forming the coating, and the raw materials do not include the dispersion medium.

According to the preparation method for the catalyst of the present invention, the first slurry and the second slurry may each independently comprise oxalic acid. A mass ratio of oxalic acid to the vanadium source in the first slurry and the second slurry may be 2-2.5:1.

According to the preparation method for the catalyst of the present invention, the first slurry and the second slurry may each independently comprise a binder. The binder is at least one selected from the group consisting of vinyl acetate-acrylate copolymer emulsion, vinyl acetate-ethylene copolymer emulsion, vinyl acetate-maleate copolymer emulsion, and acrylic acid-maleic acid copolymer emulsion. The mass percentage content of the binder may be 4-4.3% based on the total amount of the first slurry. The mass percentage content of the binder in the second slurry may be 4.3-4.6%. According to the preparation method for the catalyst of the present invention, the second slurry comprises a pore-expanding agent. The pore-expanding agent may be at least one selected from the group consisting of stearic acid and sodium stearate. The mass percentage content of the pore-expanding agent may be 3.9-4.2% based on the total amount of the second slurry. According to the preparation method for the catalyst of the present invention, the first slurry preferably does not comprise the pore-expanding agent.

According to the preparation method for the catalyst of the present invention, the first dispersion medium and the second dispersion medium each independently comprise water and an aqueous organic solvent. Preferably, the aqueous organic solvent is at least one selected from the group consisting of methanol, ethanol, formamide and N,N-dimethylformamide. According to the preparation method for the catalyst of the present invention, a mass ratio of the water to the aqueous organic solvent in the first dispersion medium and a mass ratio of the water to the aqueous organic solvent in the second dispersion medium may each independently be 1:0.1-1, preferably 1:0.3-0.8, more preferably 1:0.4-0.7.

According to the preparation method for the catalyst of the present invention, the first slurry and the second slurry may be obtained by mixing components uniformly.

In one embodiment, the first slurry may be prepared by a method including the steps of:
(1-1) mixing oxalic acid, the vanadium source, the compound containing group VA non-metallic element, the compound containing alkali metal element and the first dispersion medium to obtain a mixed solution A; and
(1-2) mixing the mixed solution A, the titanium source, the compound containing assistant metal element, and the binder to obtain the first slurry.

The mixing in the step (1-1) and the mixing in the step (1-2) each may be carried out at a temperature of 20-90°C. The mixing in the step (1-2) may be carried out in a ball mill, and a duration of ball milling may be 1-5 hours, preferably 2-4 hours.

In one embodiment, the second slurry may be prepared by a method including the steps of:
(2-1) mixing oxalic acid, the vanadium source, optionally the compound containing group VA non-metallic element, the compound containing alkali metal element and the first dispersion medium to obtain a mixed solution B; and
(2-2) mixing the mixed solution B, the titanium source, the compound containing the assistant metal element, and the binder to obtain the second slurry.

The mixing in the step (2-1) and the mixing in the step (2-2) each may be carried out at a temperature of 20-90°C. The mixing in the step (2-2) may be carried out in a ball mill, and a duration of ball milling may be 1-5 hours, preferably 2-4 hours.

According to the preparation method for the catalyst of the present invention, the carrier is an inert inorganic carrier and may be at least one selected from the group consisting of alumina, talc, silicon carbide, aluminum silicate, quartz and ceramic. According to the preparation method for the catalyst of the present invention, a shape of the carrier may be cylindrical, spherical, annular or granular. Preferably, the shape of the carrier is annular. In a preferred embodiment, the carrier is an annular carrier, and an outer diameter of the carrier (i.e., a diameter of an outer ring) may be 3-13 mm, and may be, for example, 3 mm, 4 mm, 5 mm, 6 mm, 7 mm, 8 mm, 9 mm, 10 mm, 11 mm, 12 mm or 13 mm. Preferably, the carrier has an outer diameter of 5-9 mm. In this preferred embodiment, the carrier has a wall thickness (i.e., a difference between the diameter of the outer ring and a diameter of an inner ring/2) of 0.1-10 mm, preferably 0.1-5 mm, more preferably 1-3 mm. In this preferred embodiment, a height of the carrier may be 2-12 mm, and may be, for example, 2 mm, 3 mm, 4 mm, 5 mm, 6 mm, 7 mm, 8 mm, 9 mm, 10 mm, 11 mm or 12 mm, preferably 3-8 mm.

According to the preparation method for the catalyst of the present invention, a weight ratio of the first coating to the second coating to the carrier may be 5.5-6.8:1.9-3.5:100, preferably 5.8-6.7:1.9-2.9:100. According to the preparation method for the catalyst of the present invention, the coating temperature of the first slurry is higher than the coating temperature of the second slurry. In the present invention, the coating temperature refers to the temperature of the carrier during spray coating. Preferably, the coating temperature of the first slurry is T₁, and the coating temperature of the second slurry is T₂, and T₁-T₂ (a difference between T₁ and T₂) is in the range of 70-150°C, preferably in the range of 80-120°C, more preferably in the range of 85-105°C. In a preferred embodiment, the coating temperature of the first slurry is 200-250°C, and may be, for example, 200°C, 205°C, 210°C, 215°C, 220°C, 225°C, 230°C, 235°C, 240°C, 245°C or 250°C. In another preferred embodiment, the coating temperature of the second slurry is 100-130°C, and may be, for example, 100°C, 105°C, 110°C, 115°C, 120°C, 125°C or 130°C.

According to the preparation method for the catalyst of the present invention, a first slurry layer formed on the surface of the carrier may be cured by a conventional method to form the first coating. In the present invention, the term "curing" means that a slurry layer loses its fluidity and changes from liquid to solid. In one embodiment, the first slurry layer may be dried by using a first gas stream to form the first coating. The first gas stream is at a temperature such that the first slurry layer loses fluidity to form a coating. The temperature of the first gas stream may be 200-250°C, and may be, for example, 200°C, 205°C, 210°C, 215°C, 220°C, 225°C, 230°C, 235°C, 240°C, 245°C or 250°C. According to the preparation method for the catalyst of the present invention, the surface where the first coating is formed is coated with the second slurry to form a second slurry layer, and purging may be performed directly with a purging gas after the coating is completed to form the second coating; and the second coating may also be formed by first drying with a second gas stream and then purging with a purging gas stream. The temperature of the purging gas stream is preferably 380-450 °C, and may be, for example, 380°C, 385°C, 400°C, 405°C, 410°C, 415°C, 420°C, 425°C, 430°C, 435°C, 440°C, 445°C or 450°C. The temperature of the second gas stream is preferably 100-150°C, and may be, for example, 100°C, 105°C, 110°C, 115°C, 120°C, 125°C, 130°C, 135°C, 140°C, 145°C or 150°C. According to the preparation method for the catalyst of the present invention, the first slurry and the second slurry may coat the carrier by using a conventional method. In one embodiment, the first slurry and the second slurry separately coat the carrier by means of spray coating. In this embodiment, a spray coating rate of the first slurry and a spray coating rate of the second slurry may each independently be 30-60 mL/min per 2000 grams of the carrier, and may be, for example, 30 mL/min, 35 mL/min, 40 mL/min, 45 mL/min, 50 mL/min, 55 mL/min or 60 mL/min, preferably 35-50 mL/min.

Spray coating can be carried out on a commonly used spray coating device. As an example, the spray coating device may include a hot air fan, a coating main machine, an exhaust fan, a slurry spray coating system, a control system, and a high-voltage power supply cabinet. The coating main machine may include a coating drum and a power mechanism thereof, wherein the coating drum is enclosed in the coating main machine, and a rotation speed can be adjusted. The coating drum adopts a horizontal hollow columnar structure that can improve a contact opportunity of an active component and an inert carrier material. The horizontal hollow columnar structure is provided with a circular mesh having a pore size of 1-8 mm, preferably 3-4 mm, so that hot air can enter the coating drum, and after a solvent in the slurry on the carrier is evaporated, the evaporated solvent is taken away from the coating drum, wherein a rotating speed of the coating drum is preferably 5-10 rpm. The slurry spray coating system includes a spray nozzle and a feeding system comprising a barrel, an agitator, a feed pump, and a feed delivery pipeline. The feed pump can adjust the spray coating rate, and the spray nozzle is used to evenly spray a slurry input through the feed pump onto the surface of the carrier.

According to a third aspect of the present invention, the present invention provides a catalyst prepared by the method in the second aspect of the present invention.

The catalyst prepared by the method in the second aspect of the present invention includes a carrier and a catalytically active component coating supported on the carrier, the catalytically active component coating comprises a first coating and a second coating, the first coating is close to a surface of the carrier, and the second coating is distant from the surface of the carrier, wherein in the first coating, a mass ratio of a titanium element in terms of Ti to a vanadium element in terms of V is Ti/V¹, in the second coating, a mass ratio of the titanium element in terms of Ti to the vanadium element in terms of V is Ti/V², and Ti/V²=Ti/V¹+ΔTi/V, ΔTi/V being in range of 3 to 9. Preferably, ΔTi/V is in range of 3.5 to 8.5.

According to a fourth aspect of the present invention, the present invention provides application of the catalyst according to the first aspect of the present invention or the catalyst according to the third aspect of the present invention as a catalyst for a reaction for oxidation of 1,2,4,5-tetraalkylbenzene to prepare benzene-1,2,4,5-tetracarboxylic dianhydride. 1,2,4,5-Tetraalkylbenzene is preferably 1,2,4,5-tetramethylbenzene (i.e., durene).

According to a fifth aspect of the present invention, the present invention provides a preparation method for benzene-1,2,4,5-tetracarboxylic dianhydride, comprising contacting 1,2,4,5-tetraalkylbenzene and an oxygen-containing gas with the catalyst according to the first aspect or the third aspect of the present invention to obtain a product gas stream comprising benzene-1,2,4,5-tetracarboxylic dianhydride.

According to the preparation method for benzene-1,2,4,5-tetracarboxylic dianhydride of the present invention, 1,2,4,5-tetraalkylbenzene is preferably 1,2,4,5-tetramethylbenzene (i.e., durene).

According to the preparation method for benzene-1,2,4,5-tetracarboxylic dianhydride of the present invention, the concentration of 1,2,4,5-tetraalkylbenzene may be in range of 1-50 g/m³, preferably in range of 10-30 g/m³. The concentration of 1,2,4,5-tetraalkylbenzene refers to the number of grams of 1,2,4,5-tetraalkylbenzene contained in the oxygen-containing gas per unit volume. The higher the value, the higher the content of durene in the oxygen-containing gas.

According to the preparation method for benzene-1,2,4,5-tetracarboxylic dianhydride of the present invention, the temperature at which the raw material mixed gas is contacted with the catalyst may be 300-400°C, preferably 320-360°C, more preferably 330-350°C.

According to the preparation method for benzene-1,2,4,5-tetracarboxylic dianhydride of the present invention, the raw material mixed gas containing 1,2,4,5-tetraalkylbenzene and oxygen may contact in a common reactor. In one embodiment, the reactor is a fluidized-bed reactor. In a preferred embodiment, the reactor is a fixed-bed reactor. In a more preferred embodiment, the reactor is a fixed bed single tube reactor. In this more preferred embodiment, the fixed bed single tube reactor may have a tube length of 3000-4800 mm and an inner diameter of 20-30 mm. Reaction heat can be forcibly exchanged by allowing a molten salt to circularly flow outside a reaction tube of the fixed bed single tube reactor. In this preferred embodiment, the catalyst can be in a single stage (i.e., the catalyst is loaded in one stage of the reaction tube of the fixed bed single tube reactor) or two or more stages (i.e., the catalyst is loaded in two or more stages of the reaction tube of the fixed bed single tube reactor). According to the preparation method for benzene-1,2,4,5-tetracarboxylic dianhydride of the present invention, a volume space velocity of the oxygen-containing gas may be 1500-5000 h⁻¹, preferably 2000-4000 h⁻¹.

The present invention is described in detail below with reference to examples, but the scope of the present invention is not thereby limited.

In the following examples and Comparative examples, the mass ratio of the titanium element to the vanadium element (Ti/V¹ and Ti/V²) in the first coating and the second coating of the catalyst was determined by a scanning electron microscope (HITACHI-4800) equipped with an energy dispersive spectrometer (EDAX GENESIS XM2 SYSTEM 60x), and a specific test method was as follows: a test was carried out by using an EDAX energy dispersive spectrometer (TEAM Octane Super) under the same scanning electron microscope testing conditions, after catalyst particles were cooled with liquid nitrogen, the catalyst particles were cut from one side having no coating in a longitudinal direction to obtain catalyst semi-particles with an exposed cross section, the catalyst semi-particles were fixed to a conductive tape, and gold, platinum or carbon was sprayed onto the surfaces to increase their conductivity to obtain a test sample; and the test sample was placed in a sample stage of the scanning electron microscope, the cross section of the catalyst semi-particles was observed by using the scanning electron microscope, and the mass ratio of Ti/V of the coating at different thicknesses (also referred to as the "longitudinal direction") was measured by using the energy dispersive spectrometer, wherein a method for determining the mass ratio of Ti/V at the same coating thickness was as follows: three immediately adjacent points were randomly selected along a horizontal direction of the coating (i.e., a direction perpendicular or substantially perpendicular to a thickness direction of the coating), and magnified by the same factor (typically, the magnification factor is 500-1000X), the mass ratio of Ti/V and the vanadium content and the titanium content at the point were measured by using the energy dispersive spectrometer, and values measured at the three points were averaged as the mass ratio of Ti/V in the horizontal direction. The first coating and the second coating are distinguished by a difference of the mass ratio of Ti/V between two adjacent points in the thickness direction of the coating, if the difference of the mass ratio of Ti/V between two adjacent points is within the range of ΔTi/V defined in the present invention, then the two points belong to the first coating and the second coating; and if the difference of the mass ratio of Ti/V between two adjacent points is less than the range ΔTi/V defined in the present invention (including two endpoint values), then the two points belong to the same coating. Test conditions for the energy dispersive spectrometer included: an excitation source being a high energy electron beam with an energy of 15 kv.

In the following examples and Comparative examples, the total pore volume of the catalyst was determined by the BET specific surface method on a fully automated physicochemical adsorption analyzer purchased from Micromeritics, Inc., USA, and a specific test method was as follows: a quantitative sample was put into a sample tube, and the sample tube was subjected to degassing under the conditions of increasing the temperature at 10°C/min to 350°C, and vacuuming for 4 h; then the sample tube was then placed in the analyzer for full analysis of an isotherm of a mesoporous material, and a specific surface area of the catalyst was calculated by the BET method.

In the following examples and Comparative examples, preparation of a slurry was carried out at room temperature (25°C).

In the following examples and Comparative examples, titanium dioxide is anatase titanium dioxide having a specific surface area in the range of 20-30 m²/g.

### Examples 1-4 were used to prepare the catalyst of the present invention.

### Example 1

### 1. Preparation of a first slurry

(1-i) 123.23 g of ammonium metavanadate, 275.36 g of oxalic acid, 2.11 g of cesium sulfate, 10.23 g of ammonium dihydrogen phosphate, formamide and 350 g of water were mixed to prepare a solution A-1, wherein a weight ratio of the formamide to the water was 0.7:1.
(1-ii) The solution A-1, 630 g of titanium dioxide, 6.12 g of niobium oxalate, 7.24 g of zirconium sulfate and 23.1 g of antimony trioxide were poured into a ball mill, then 70 g of a vinyl acetate/ethylene copolymer emulsion was added, and ball milling was performed for 4 h to obtain the first slurry.

### 2. Preparation of a second slurry

(2-i) 69.82 g of ammonium metavanadate, 161.3 g of oxalic acid, 4.57 g of cesium sulfate, formamide and 350 g of water were mixed to prepare a solution B-1, wherein a weight ratio of the formamide to the water was 0.7:1.
(2-ii) The solution B-1, 630 g of titanium dioxide, 4.86 g of niobium oxalate, 13.96 g of antimony trioxide and 63 g of stearic acid were poured into a ball mill, then 70 g of a vinyl acetate/ethylene copolymer emulsion was added, and ball milling was performed for 4 h to obtain the second slurry.

### 3. Preparation of a catalyst

(1-iii) 2000 g of a carrier porcelain ring (having an outer diameter of 8 mm, a height of 6 mm, and a wall thickness of 1.5 mm, and being a talc ring) was placed in a drum, a speed of the drum being controlled to be 10 rpm; the first slurry was added into a stirring tank of a feed liquid spray coating system to be stirred; a hot air fan was turned on to make hot air of 100°C pass through the drum to preheat the carrier porcelain ring, and when the temperature of the carrier porcelain ring reached 210°C, a feed spray nozzle was opened, a spray coating rate of the first slurry being controlled to be 30 mL/min; the first slurry was spray-coated on the surface of the carrier through the spray nozzle, and dried rapidly via hot air of 220°C; and when the content of an inner coating reached 6.2 wt% of the weight of the carrier, spray coating was completed to obtain a catalyst intermediate.
(2-iii) The second slurry was added into the stirring tank of the feed liquid spray coating system to be stirred; the hot air fan was turned on to make hot air of 100°C pass through the drum, when the temperature of the carrier reached 120°C, the feed spray nozzle was opened, a spray coating rate of the second slurry being controlled to be 30 mL/min, and the second slurry was spray-coated on the surface of the catalyst intermediate through the spray nozzle, and rapidly dried via hot air of 120°C; and when the content of an outer coating reached 2.3 wt% of the weight of the carrier, spray coating was completed, and purging was performed with hot air of 400°C for 2 h to obtain a catalyst S1, wherein the composition and the total pore volume of the catalyst S1 are listed in Table 1. Wherein the catalyst S1 comprises a carrier, and a first coating (i.e., the inner coating) and a second coating (i.e., the outer coating) which are sequentially supported on the carrier, and in the catalyst S1, a weight ratio of the first coating to the second coating to the carrier was 6.2:2.3:100.

### Example 2

### 1. Preparation of a first slurry

(1-i) 123.23 g of ammonium metavanadate, 275.36 g of oxalic acid, 2.11 g of cesium sulfate, 10.23 g of ammonium dihydrogen phosphate, formamide and 350 g of water were mixed to prepare a solution A-1, wherein a weight ratio of the formamide to the water was 0.7:1.
(1-ii) The solution A-1, 630 g of titanium dioxide, 6.12 g of niobium oxalate, 7.24 g of zirconium sulfate and 23.1 g of antimony trioxide were poured into a ball mill, then 70 g of a vinyl acetate/ethylene copolymer emulsion was added, and ball milling was performed for 4 h to obtain the first slurry.

### 2. Preparation of a second slurry

(2-i) 57.31 g of ammonium metavanadate, 123.89 g of oxalic acid, 3.77 g of cesium sulfate, formamide and 350 g of water were mixed to prepare a solution B-2, wherein a weight ratio of the formamide to the water was 0.7:1.
(2-ii) The solution B-2, 630 g of titanium dioxide, 3.92 g of niobium oxalate, 11.29 g of antimony trioxide and 63 g of stearic acid were poured into a ball mill, then 70 g of a vinyl acetate/ethylene copolymer emulsion was added, and ball milling was performed for 4 h to obtain the second slurry.

### 3. Preparation of a catalyst

(1-iii) 2000 g of a carrier porcelain ring (having an outer diameter of 8 mm, a height of 6 mm, and a wall thickness of 1.5 mm, and being a talc ring) was placed in a drum, a speed of the drum being controlled to be 10 rpm; the first slurry was added into a stirring tank of a feed liquid spray coating system to be stirred; a hot air fan was turned on to make hot air of 100°C pass through the drum to preheat the carrier porcelain ring, and when the temperature of the carrier porcelain ring reached 210°C, a feed spray nozzle was opened, a spray coating rate of the first slurry being controlled to be 30 mL/min; the first slurry was spray-coated on the surface of the carrier through the spray nozzle, and dried rapidly via hot air of 220°C; and when the content of an inner coating reached 5.8 wt% of the weight of the carrier, spray coating was completed to obtain a catalyst intermediate.
(2-iii) The second slurry was added into the stirring tank of the feed liquid spray coating system to be stirred; the hot air fan was turned on to make hot air of 100°C pass through the drum, when the temperature of the carrier reached 120°C, the feed spray nozzle was opened, a spray coating rate of the second slurry being controlled to be 30 mL/min, and the second slurry was spray-coated on the surface of the catalyst intermediate through the spray nozzle, and rapidly dried via hot air of 120°C; and when the content of an outer coating reached 1.9 wt% of the weight of the carrier, spray coating was completed, and purging was performed with hot air of 400°C for 2 h to obtain a catalyst S2, wherein the composition and the total pore volume of the catalyst S2 are listed in Table 1. Wherein the catalyst S2 comprises a carrier, and a first coating (i.e., the inner coating) and a second coating (i.e., the outer coating) which are sequentially supported on the carrier, and in the catalyst S2, a weight ratio of the first coating to the second coating to the carrier was 5.8:1.9:100.

### Example 3

### 1. Preparation of a first slurry

(1-i) 102.55 g of ammonium metavanadate, 230.27 g of oxalic acid, 1.72 g of cesium sulfate, 8.76 g of ammonium dihydrogen phosphate, formamide and 350 g of water were mixed to prepare a solution A-3, wherein a weight ratio of the formamide to the water was 0.7:1.
(1-ii) The solution A-3, 630 g of titanium dioxide, 5.83 g of niobium oxalate, 7.15 g of zirconium sulfate and 21.8 g of antimony trioxide were poured into a ball mill, then 70 g of a vinyl acetate/ethylene copolymer emulsion was added, and ball milling was performed for 4 h to obtain the first slurry.

### 2. Preparation of a second slurry

(2-i) 69.82 g of ammonium metavanadate, 161.3 g of oxalic acid, 4.57 g of cesium sulfate, formamide and 350 g of water were mixed to prepare a solution B-1, wherein a weight ratio of the formamide to the water was 0.7:1.
(2-ii) The solution B-1, 630 g of titanium dioxide, 4.86 g of niobium oxalate, 13.96 g of antimony trioxide and 63 g of stearic acid were poured into a ball mill, then 70 g of a vinyl acetate/ethylene copolymer emulsion was added, and ball milling was performed for 4 h to obtain the second slurry.

### 3. Preparation of a catalyst

(1-iii) 2000 g of a carrier porcelain ring (having an outer diameter of 8 mm, a height of 6 mm, and a wall thickness of 1.5 mm, and being a talc ring) was placed in a drum, a speed of the drum being controlled to be 10 rpm; the first slurry was added into a stirring tank of a feed liquid spray coating system to be stirred; a hot air fan was turned on to make hot air of 100°C pass through the drum to preheat the carrier porcelain ring, and when the temperature of the carrier porcelain ring reached 210°C, a feed spray nozzle was opened, a spray coating rate of the first slurry being controlled to be 30 mL/min; the first slurry was spray-coated on the surface of the carrier through the spray nozzle, and dried rapidly via hot air of 220°C; and when the content of an inner coating reached 6.5 wt% of the weight of the carrier, spray coating was completed to obtain a catalyst intermediate.
(2-iii) The second slurry was added into the stirring tank of the feed liquid spray coating system to be stirred; the hot air fan was turned on to make hot air of 100°C pass through the drum, when the temperature of the carrier reached 120°C, the feed spray nozzle was opened, a spray coating rate of the second slurry being controlled to be 30 mL/min, and the second slurry was spray-coated on the surface of the catalyst intermediate through the spray nozzle, and rapidly dried via hot air of 120°C; and when the content of an outer coating reached 2.7 wt% of the weight of the carrier, spray coating was completed, and purging was performed with hot air of 400°C for 2 h to obtain a catalyst S3, wherein the composition and the total pore volume of the catalyst S3 are listed in Table 1. Wherein the catalyst S3 comprises a carrier, and a first coating (i.e., the inner coating) and a second coating (i.e., the outer coating) which are sequentially supported on the carrier, and in the catalyst S3, a weight ratio of the first coating to the second coating to the carrier was 6.5:2.7:100.

### Example 4

### 1. Preparation of a first slurry

(1-i) 102.55 g of ammonium metavanadate, 230.27 g of oxalic acid, 1.72 g of cesium sulfate, 8.76 g of ammonium dihydrogen phosphate, formamide and 350 g of water were mixed to prepare a solution A-3, wherein a weight ratio of the formamide to the water was 0.7:1.
(1-ii) The solution A-3, 630 g of titanium dioxide, 5.83 g of niobium oxalate, 7.15 g of zirconium sulfate and 21.8 g of antimony trioxide were poured into a ball mill, then 70 g of a vinyl acetate/ethylene copolymer emulsion was added, and ball milling was performed for 4 h to obtain the first slurry.

### 2. Preparation of a second slurry

(2-i) 57.31 g of ammonium metavanadate, 123.89 g of oxalic acid, 3.77 g of cesium sulfate, formamide and 350 g of water were mixed to prepare a solution B-2, wherein a weight ratio of the formamide to the water was 0.7:1.
(2-ii) The solution B-2, 630 g of titanium dioxide, 3.92 g of niobium oxalate, 11.29 g of antimony trioxide and 63 g of stearic acid were poured into a ball mill, then 70 g of a vinyl acetate/ethylene copolymer emulsion was added, and ball milling was performed for 4 h to obtain the second slurry.

### 3. Preparation of a catalyst

(1-iii) 2000 g of a carrier porcelain ring (having an outer diameter of 8 mm, a height of 6 mm, and a wall thickness of 1.5 mm, and being a talc ring) was placed in a drum, a speed of the drum being controlled to be 10 rpm; the first slurry was added into a stirring tank of a feed liquid spray coating system to be stirred; a hot air fan was turned on to make hot air of 100°C pass through the drum to preheat the carrier porcelain ring, and when the temperature of the carrier porcelain ring reached 210°C, a feed spray nozzle was opened, a spray coating rate of the first slurry being controlled to be 30 mL/min; the first slurry was spray-coated on the surface of the carrier through the spray nozzle, and dried rapidly via hot air of 220°C; and when the content of an inner coating reached 6.7 wt% of the weight of the carrier, spray coating was completed to obtain a catalyst intermediate.
(2-iii) The second slurry was added into the stirring tank of the feed liquid spray coating system to be stirred; the hot air fan was turned on to make hot air of 100°C pass through the drum, when the temperature of the carrier reached 120°C, the feed spray nozzle was opened, a spray coating rate of the second slurry being controlled to be 30 mL/min, and the second slurry was spray-coated on the surface of the catalyst intermediate through the spray nozzle, and rapidly dried via hot air of 120°C; and when the content of an outer coating reached 1.9 wt% of the weight of the carrier, spray coating was completed, and purging was performed with hot air of 450°C for 2 h to obtain a catalyst S4, wherein the composition and the total pore volume of the catalyst S4 are listed in Table 1. Wherein the catalyst S4 comprises a carrier, and a first coating (i.e., the inner coating) and a second coating (i.e., the outer coating) which are sequentially supported on the carrier, and in the catalyst S4, a weight ratio of the first coating to the second coating to the carrier was 6.7:1.9: 100.

### Comparative Example 1

A catalyst was prepared by the same method as that in Example 1, except that in Comparative example 1, a second slurry was not prepared, and the prepared catalyst did not comprise a second coating, and specific operations were as follows: a first slurry was prepared by the same method as that in Example 1, 2000 g of a carrier porcelain ring (the same as that in Example 1) was placed in a drum, a speed of the drum being controlled to be 10 rpm; the first slurry was added into a stirring tank of a feed liquid spray coating system to be stirred; a hot air fan was turned on to make hot air of 100°C pass through the drum to preheat the carrier porcelain ring, and when the temperature of the carrier porcelain ring reached 210°C, a feed spray nozzle was opened, a spray coating rate of the first slurry being controlled to be 30 mL/min; the first slurry was spray-coated on the surface of the carrier through the spray nozzle, and dried rapidly via hot air of 220°C; and when the content of an inner coating reached 8.3 wt% of the weight of the carrier, spray coating was completed to obtain a catalyst DS 1. Wherein the catalyst DS1 comprises a carrier and a coating supported on the carrier; and in the catalyst DS 1, a weight ratio of the coating to the carrier was 8.3:100.

### Comparative Example 2

A catalyst was prepared by the same method as that in Example 1, except that in Comparative example 2, a first slurry was not prepared, the prepared catalyst did not comprise a first coating, but a second slurry prepared by the same method as that in Example 1 was directly spray-coated on the surface of the carrier to form a coating, and specific operations were as follows: the second slurry was prepared by the same method as that in Example 1, 2000 g of a carrier porcelain ring (the same as that in Example 1) was placed in a drum, a speed of the drum being controlled to be 10 rpm; the first slurry was added into a stirring tank of a feed liquid spray coating system to be stirred; a hot air fan was turned on to make hot air of 100°C pass through the drum to preheat the carrier porcelain ring, and when the temperature of the carrier porcelain ring reached 210°C, a feed spray nozzle was opened, a spray coating rate of the first slurry being controlled to be 30 mL/min; the first slurry was spray-coated on the surface of the carrier through the spray nozzle, and dried rapidly via hot air of 220°C; and when the content of an inner coating reached 8.8 wt% of the weight of the carrier, spray coating was completed to obtain a catalyst DS2. Wherein the catalyst DS2 comprises a carrier and a coating supported on the carrier; and in the catalyst DS2, a weight ratio of the coating to the carrier was 8.8:100.

### Comparative Example 3

A catalyst was prepared by the same method as that in Example 2, except that in Comparative example 3, a first slurry was not prepared, the prepared catalyst did not comprise a first coating, but a second slurry prepared by the same method as that in Example 2 was directly spray-coated on the surface of the carrier to form a coating, and specific operations were as follows: the second slurry was prepared by the same method as that in Example 2, 2000 g of a carrier porcelain ring (the same as that in Example 2) was placed in a drum, a speed of the drum being controlled to be 10 rpm; the second slurry was added into a stirring tank of a feed liquid spray coating system to be stirred; a hot air fan was turned on to make hot air of 100°C pass through the drum to preheat the carrier porcelain ring, and when the temperature of the carrier porcelain ring reached 210°C, a feed spray nozzle was opened, a spray coating rate of the second slurry being controlled to be 30 mL/min; the first second was spray-coated on the surface of the carrier through the spray nozzle, and dried rapidly via hot air of 220°C; and when the content of an inner coating reached 8.7 wt% of the weight of the carrier, spray coating was completed to obtain a catalyst DS3. Wherein the catalyst DS3 comprises a carrier and a coating supported on the carrier; and in the catalyst DS3, a weight ratio of the coating to the carrier was 8.7:100.

### Comparative Example 4

### 1. Preparation of a first slurry

(1-i) 102.55 g of ammonium metavanadate, 230.27 g of oxalic acid, 1.72 g of cesium sulfate, 8.76 g of ammonium dihydrogen phosphate, formamide and 350 g of water were mixed to prepare a solution A-3, wherein a weight ratio of the formamide to the water was 0.7:1.
(1-ii) The solution A-3, 630 g of titanium dioxide, 5.83 g of niobium oxalate, 7.15 g of zirconium sulfate and 21.8 g of antimony trioxide were poured into a ball mill, then 70 g of a vinyl acetate/ethylene copolymer emulsion was added, and ball milling was performed for 4 h to obtain the first slurry.

### 2. Preparation of a second slurry

(2-i) 90.28 g of ammonium metavanadate, 218.12 g of oxalic acid, 5.97 g of cesium sulfate, formamide and 350 g of water were mixed to prepare a solution B-4, wherein a weight ratio of the formamide to the water was 0.7:1.
(2-ii) The solution B-4, 630 g of titanium dioxide, 6.51 g of niobium oxalate, 17.85 g of antimony trioxide and 63 g of stearic acid were poured into a ball mill, then 70 g of a vinyl acetate/ethylene copolymer emulsion was added, and ball milling was performed for 4 h to obtain the second slurry.

### 3. Preparation of a catalyst

(1-iii) 2000 g of a carrier porcelain ring (the same as that in Example 3) was placed in a drum, a speed of the drum being controlled to be 10 rpm; the first slurry was added into a stirring tank of a feed liquid spray coating system to be stirred; a hot air fan was turned on to make hot air of 100°C pass through the drum to preheat the carrier porcelain ring, and when the temperature of the carrier porcelain ring reached 210°C, a feed spray nozzle was opened, a spray coating rate of the first slurry being controlled to be 30 mL/min; the first slurry was spray-coated on the surface of the carrier through the spray nozzle, and dried rapidly via hot air of 220°C; and when the content of an inner coating reached 6.8 wt% of the weight of the carrier, spray coating was completed to obtain a catalyst intermediate.
(2-iii) The second slurry was added into the stirring tank of the feed liquid spray coating system to be stirred; the hot air fan was turned on to make hot air of 100°C pass through the drum, when the temperature of the carrier reached 120°C, the feed spray nozzle was opened, a spray coating rate of the second slurry being controlled to be 30 mL/min, and the second slurry was spray-coated on the surface of the catalyst intermediate through the spray nozzle, and rapidly dried via hot air of 120°C; and when the content of an outer coating reached 1.9 wt% of the weight of the carrier, spray coating was completed, and purging was performed with hot air of 400°C for 2 h to obtain a catalyst DS4, wherein the composition and the total pore volume of the catalyst DS4 are listed in Table 1. Wherein the catalyst DS4 comprises a carrier, and a first coating (i.e., the inner coating) and a second coating (i.e., the outer coating) which are sequentially supported on the carrier, and in the catalyst DS4, a weight ratio of the first coating to the second coating to the carrier was 6.8:1.9:100.

### Comparative Example 5

### 1. Preparation of a first slurry

(1-i) 102.55 g of ammonium metavanadate, 230.27 g of oxalic acid, 1.72 g of cesium sulfate, 8.76 g of ammonium dihydrogen phosphate, formamide and 350 g of water were mixed to prepare a solution A-3, wherein a weight ratio of the formamide to the water was 0.7:1.
(1-ii) The solution A-3, 630 g of titanium dioxide, 5.83 g of niobium oxalate, 7.15 g of zirconium sulfate and 21.8 g of antimony trioxide were poured into a ball mill, then 70 g of a vinyl acetate/ethylene copolymer emulsion was added, and ball milling was performed for 4 h to obtain the first slurry.

### 2. Preparation of a second slurry

(2-i) 40.19 g of ammonium metavanadate, 84.32 g of oxalic acid, 2.33 g of cesium sulfate, formamide and 350 g of water were mixed to prepare a solution B-5, wherein a weight ratio of the formamide to the water was 0.7:1.
(2-ii) The solution B-5, 630 g of titanium dioxide, 2.48 g of niobium oxalate, 7.32 g of antimony trioxide and 63 g of stearic acid were poured into a ball mill, then 70 g of a vinyl acetate/ethylene copolymer emulsion was added, and ball milling was performed for 4 h to obtain the second slurry.

### 3. Preparation of a catalyst

(1-iii) 2000 g of a carrier porcelain ring (the same as that in Example 3) was placed in a drum, a speed of the drum being controlled to be 10 rpm; the first slurry was added into a stirring tank of a feed liquid spray coating system to be stirred; a hot air fan was turned on to make hot air of 100°C pass through the drum to preheat the carrier porcelain ring, and when the temperature of the carrier porcelain ring reached 210°C, a feed spray nozzle was opened, a spray coating rate of the first slurry being controlled to be 30 mL/min; the first slurry was spray-coated on the surface of the carrier through the spray nozzle, and dried rapidly via hot air of 220°C; and when the content of an inner coating reached 6.9 wt% of the weight of the carrier, spray coating was completed to obtain a catalyst intermediate.
(2-iii) The second slurry was added into the stirring tank of the feed liquid spray coating system to be stirred; the hot air fan was turned on to make hot air of 100°C pass through the drum, when the temperature of the carrier reached 120°C, the feed spray nozzle was opened, a spray coating rate of the second slurry being controlled to be 30 mL/min, and the second slurry was spray-coated on the surface of the catalyst intermediate through the spray nozzle, and rapidly dried via hot air of 120°C; and when the content of an outer coating reached 2.1 wt% of the weight of the carrier, spray coating was completed, and purging was performed with hot air of 400°C for 2 h to obtain a catalyst DS5, wherein the composition and the total pore volume of the catalyst DS5 are listed in Table 1. Wherein the catalyst DS5 comprises a carrier, and a first coating (i.e., the inner coating) and a second coating (i.e., the outer coating) which are sequentially supported on the carrier, and in the catalyst DS5, a weight ratio of the first coating to the second coating to the carrier was 6.9:2.1:100.

### Comparative Example 6

A catalyst was prepared by the same method as that in Example 1, except that the second slurry was used in (1-iii) of the step 3, and the first slurry was used in the step (2-iii), i.e., the second slurry was used to form the first coating (i.e., the inner coating) and the first slurry was used to form the second coating (i.e., the outer coating) to obtain a catalyst DS6, wherein the composition and the total pore volume of the catalyst DS6 are listed in Table 1. Wherein the catalyst DS6 comprises a carrier, and a first coating (i.e., the inner coating) and a second coating (i.e., the outer coating) which are sequentially supported on the carrier, and in the catalyst DS6, a weight ratio of the first coating to the second coating to the carrier was 6.2:2.3:100.

**Table 1**

| Catalyst | Ti/V¹ | Ti/V² | ΔTi/V | Ti (wt%) | | P (wt%) | Cs (wt%) | | Assistant (wt%) | | Total pore volume (cm³/g) |
|---|---|---|---|---|---|---|---|---|---|---|---|
| | | | | First coating | Second coating | First coating | First coating | Second coating | First coating | Second coating | |
| S1 | 7.03 | 12.32 | 5.29 | 49.8 | 53.7 | 0.36 | 0.2 | 0.48 | 3.66 | 2.16 | 0.046 |
| S2 | 6.88 | 15.14 | 8.26 | 49.8 | 54.8 | 0.36 | 0.2 | 0.4 | 3.66 | 1.78 | 0.038 |
| S3 | 8.46 | 12.22 | 3.76 | 51.1 | 53.7 | 0.32 | 0.17 | 0.48 | 3.56 | 2.16 | 0.051 |
| S4 | 8.47 | 15.39 | 6.92 | 51.1 | 54.8 | 0.32 | 0.17 | 0.4 | 3.56 | 1.78 | 0.034 |
| DS4 | 8.56 | 9.61 | 1.05 | 51.1 | 52.2 | 0.32 | 0.17 | 0.61 | 3.56 | 2.69 | 0.038 |
| DS5 | 8.64 | 21.57 | 12.93 | 51.1 | 56.3 | 0.32 | 0.17 | 0.26 | 3.56 | 1.18 | 0.041 |
| DS6 | 12.22 | 7.01 | -5.21 | 53.7 | 49.8 | 0 | 0.48 | 0.2 | 2.16 | 3.66 | 0.036 |

| | | | | | | | | | | | |
|---|---|---|---|---|---|---|---|---|---|---|---|
| 1: A mass ratio of titanium/vanadium (Ti/V¹) in the first coating and a mass ratio of titanium/vanadium (Ti/V²) in the second coating were determined by a scanning electron microscope-energy dispersive spectroscopy, wherein Ti/V¹ is the mass ratio of the titanium element in terms of Ti to the vanadium element in terms of V in the first coating, Ti/V² is the mass ratio of the titanium element in terms of Ti to the vanadium element in terms of V in the second coating, and Ti/V²=Ti/V¹+ΔTi/V; 2: The contents of titanium (Ti), phosphorus (P), cesium (Cs) and the assistant were calculated by the feeding amount on the basis of the total amount (in terms of oxides) of raw materials forming the coating, and the raw materials do not include dispersion medium, the titanium (Ti) content being in terms of the titanium element, the phosphorus (P) content being in terms of P, the cesium (Cs) content being in terms of Cs, and the assistant content being in terms of an oxide. | | | | | | | | | | | |

### Test Examples and Test Comparative Examples

The performance of the catalyst was evaluated by using a fixed bed single tube reactor under simulated industrial production conditions, the fixed bed single tube reactor has an inner diameter of 29 mm, and a tube length of 4400 mm, and molten salt circulation was used to transfer heat outside a reaction tube. An outlet of the reactor is connected to an analytical system and a reactant collection system.

The catalyst adopted a single-stage (i.e., single-tube single-stage) or double-stage (i.e., single-tube double-stage) loading mode, and a total loading height of the catalyst was 2000 mm; in the double-stage loading, a first stage was loaded at an inlet end of the reaction tube and a second stage was loaded at an outlet end of the reaction tube, a loading height of the first stage was 1200 mm and a loading height of the second stage was 800 mm.

In Test Examples and Test Comparative Examples, the catalysts were respectively activated in an oxidizing atmosphere at a temperature of 410°C for 4 h before the reaction.

In Test Examples and Test Comparative Examples, a method of a gas-phase oxidation reaction was the same, including that the pressure of the reaction was atmospheric pressure (i.e., 1 atm), air was used as an oxygen-containing gas, the air has a space velocity of 2000 h⁻¹, the durene feeding concentration was increased stepwise during the reaction, and sampling analysis for each condition was performed at the outlet of the reactor to evaluate the catalyst maximum loading (i.e., the highest durene feeding concentration, that is, the durene concentration listed in Table 2) as well as a durene conversion rate and the homoanhydride yield at the molten salt temperature and maximum loading listed in Table 2. The test results are listed in Table 2.

**Table 2**

| | Catalyst source | Catalyst loading mode | Molten salt temperature, °C | Durene concentration, g/m³ | Durene conversion rate, % | Homoanhydride yield, % |
|---|---|---|---|---|---|---|
| Test Example 1 | S1 | Single-tube single-stage | 341 | 17.5 | 99.6 | 95.9 |
| Test Example 2 | S2 | Single-tube single-stage | 343 | 12.3 | 99.7 | 89.9 |
| Test Example 3 | S3 | Single-tube single-stage | 341 | 16.1 | 99.9 | 91.2 |
| Test Example 4 | S4 | Single-tube single-stage | 339 | 10.7 | 99.8 | 84.6 |
| Test Example 5 | S1+S4 | Single-tube double-stage, loading S1 at the inlet end, and loading S4 at the outlet end | 338 | 26.6 | 99.9 | 106.9 |
| Test Comparative Example 1 | DS1 | Single-tube single-stage | 341 | 8.8 | 99.8 | 76.3 |
| Test Comparative Example 2 | DS2 | Single-tube single-stage | 345 | 20.2 | 99.5 | 90.2 |
| Test Comparative Example 3 | DS2+DS3 | Single-tube double-stage, loading DS2 at the inlet end, and loading DS3 at the outlet end | 339 | 22.7 | 99.6 | 101.4 |
| Test Comparative Example 4 | DS4 | Single-tube single-stage | 340 | 6.3 | 99.8 | 79.2 |
| Test Comparative Example 5 | DS5 | Single-tube single-stage | 346 | 12.4 | 99.7 | 72.6 |
| Test Comparative Example 6 | DS6 | Single-tube single-stage | 343 | 19.9 | 99.6 | 74.5 |

By comparing Test Example 1 with Test Comparative Examples 1 and 2, it can be seen that in Test Example 1, the homoanhydride yield can be effectively increased by using the catalyst in which the first coating and the second coating are formed according to the present invention compared with forming a single-layer catalytically active coating on the surface of the carrier. By comparing Test Example 1 with Test Comparative Example 6, it can be seen that the homoanhydride yield can be effectively increased by forming the first coating and the second coating on the surface of the carrier in the manner of the present invention.

By comparing Test Example 3 with Test Comparative Examples 4 and 5, it can be seen that the catalyst activity can be effectively improved, and significantly improved homoanhydride selectivity can be obtained by controlling the mass ratio of the titanium content in terms of Ti to the vanadium content in terms of V in the first coating and the second coating to be within the range defined in the present invention.

By comparing Test Example 5 with Test Comparative Example 3, it can be seen that the catalyst according to the present invention can not only operate at a higher durene concentration, but also improve the durene conversion rate and homoanhydride selectivity when the catalyst is loaded in two stages of a single reaction tube.

## Claims

1. A catalyst for gas-phase oxidation of 1,2,4,5-tetraalkylbenzene, comprising a carrier and a catalytically active component coating supported on the carrier, wherein the catalytically active component coating comprises a first coating and a second coating, the first coating is close to a surface of the carrier, the second coating is distant from the surface of the carrier, the first coating and the second coating each independently comprise vanadium element and titanium element, a mass ratio of the titanium element in terms of Ti to the vanadium element in terms of V in the first coating is Ti/V¹, a mass ratio of the titanium element in terms of Ti to the vanadium element in terms of V in the second coating is Ti/V², and Ti/V²=Ti/V¹+ΔTi/V, ΔTi/V being in the range of 3 to 9.

2. The catalyst according to claim 1, wherein the mass ratio Ti/V¹ of the titanium element in terms of Ti to the vanadium element in terms of V in the first coating is 7-8.5: 1.

3. The catalyst according to claim 1, wherein the mass ratio Ti/V² of the titanium element in terms of Ti to the vanadium element in terms of V in the second coating is 12-15.5: 1.

4. The catalyst according to any one of claims 1 to 3, wherein the mass percentage content C_{Ti}¹ of the titanium element in the first coating in terms of Ti is in the range of 40-60%; and/or
the mass percentage content C_{Ti}² of the titanium element in the second coating in terms of Ti is in the range of 45-65%.

5. The catalyst according to any one of claims 1 to 4, wherein the catalytically active component coating further comprises at least one selected from the group consisting of group VA non-metallic element, alkali metal element and assistant metal element, and the assistant metal element is at least one selected from the group consisting of rare earth element, group VIB element, group VIII element, group IIIA metal element, group VA metal element, and group IVB element other than titanium element;
preferably, the group VA non-metallic element is phosphorus;
preferably, the alkali metal element is cesium;
preferably, the assistant metal element is at least one selected from the group consisting of niobium, zirconium and antimony.

6. The catalyst according to any one of claims 1 to 5, wherein the second coating is attached to a surface of the first coating;
preferably, the first coating is attached to the surface of the carrier.

7. The catalyst according to any one of claims 1 to 6, wherein the catalyst has a total pore volume of 0.03-0.08 mL/g.

8. The catalyst according to any one of claims 1 to 7, wherein the carrier is at least one selected from the group consisting of alumina, talc, silicon carbide, aluminum silicate, quartz, and ceramic.

9. A method for preparing a catalyst for gas-phase oxidation of 1,2,4,5-tetraalkylbenzene, comprising the steps of:
(1) coating a surface of a carrier with a first slurry to form a first coating, the first slurry comprising a first dispersion medium, a first vanadium source and a first titanium source; and
(2) coating the surface of the carrier where the first coating is formed with a second slurry to form a second coating, the second slurry comprising a second dispersion medium, a second vanadium source, a second titanium source, and a pore-expanding agent;
wherein, a mass of the vanadium source introduced in the first coating is C_{V}¹², a mass of the titanium source introduced in the first coating is C_{Ti}¹², a mass of the vanadium source introduced in the second coating is Cᵥ²², a mass of the titanium source introduced in the second coating is C_{Ti}²², Ti/V¹²=C_{Ti}¹²/C_{V}¹², Ti/V²²=C_{Ti}²²/C_{V}²², and Ti/V²²=Ti/V¹²+ΔTi/V², ΔTi/V² being in the range of 3 to 9, the vanadium source being in terms of V, and the titanium source being in terms of Ti.

10. The method according to claim 9, wherein the mass percentage content C_{Ti}¹² of the titanium source introduced in the first coating in terms of Ti is in the range of 40-60%; or
the mass percentage content C_{Ti}²² of the titanium source introduced in the second coating in terms of Ti is in the range of 45-65%.

11. The method according to claim 9 or 10, wherein the first vanadium source and the second vanadium source are each independently at least one selected from the group consisting of ammonium metavanadate, vanadium pentoxide, and sodium vanadate;
preferably, the first titanium source and the second titanium source are each independently at least one selected from the group consisting of titanium dioxide and metatitanic acid;
preferably, the carrier is at least one selected from the group consisting of alumina, talc, silicon carbide, aluminum silicate, quartz and ceramic.

12. The method according to any one of claims 9 to 11, wherein a weight ratio of the first coating to the second coating to the carrier is 5.5-6.8:1.9-3.5:100.

13. The method according to any one of claims 9 to 12, wherein the first slurry further comprises at least one compound selected from the group consisting of a compound containing group VA non-metallic element, a compound containing kali metal element and a compound containing assistant metal element, and the assistant metal element is at least one element selected from the group consisting of rare earth element, group VIB element, group VIII element, group IIIA metal element, group VA metal element, and group IVB element other than a titanium element;
preferably, the mass percentage content of the compound containing group VA non-metallic element introduced in the first coating is 0.1-0.5%, the compound containing group VA non-metallic element being in terms of an element;
preferably, the mass percentage content of the compound containing alkali metal element introduced in the first coating is 0.1-0.3%, the compound containing alkali metal element being in terms of an element;
preferably, the mass percentage content of the compound containing assistant metal element introduced in the first coating is 1-6%, the compound containing assistant metal element being in terms of an oxide;
preferably, the group VA non-metallic element is phosphorus, the alkali metal element is cesium, and the assistant metal element is at least one element selected from the group consisting of niobium, zirconium and antimony;
preferably, the compound containing group VA non-metallic element is at least one selected from the group consisting of ammonium dihydrogen phosphate, triammonium phosphate, and phosphorus pentoxide;
preferably, the compound containing alkali metal element is at least one selected from the group consisting of MNO₃, M₂SO₄, MCl and M₂CO₃, M being the alkali metal element;
Preferably, the compound containing assistant metal element is at least one selected from the group consisting of oxide of the assistant metal and water-soluble salt containing the assistant metal.

14. The method according to any one of claims 9 to 13, wherein the second slurry further comprises a compound containing alkali metal element, a compound containing assistant metal element, and optionally a compound containing group VA non-metallic element, and the assistant metal element is at least one selected from the group consisting of rare earth element, group VIB element, group VIII element, group IIIA metal element, group VA metal element, and group IVB element other than titanium element;
preferably, the mass percentage content of the compound containing alkali metal element introduced in the second coating is 0.1-0.7%, the compound containing alkali metal element being in terms of an element;
preferably, the mass percentage content of the compound containing assistant metal element introduced in the second coating is 1-6%, the compound containing assistant metal element being in terms of an oxide;
preferably, the group VA non-metallic element is phosphorus, the alkali metal element is cesium, and the assistant metal element is at least one selected from the group consisting of niobium, zirconium and antimony;
preferably, the compound containing group VA non-metallic element is at least one selected from the group consisting of ammonium dihydrogen phosphate, triammonium phosphate, and phosphorus pentoxide;
preferably, the compound containing alkali metal element is at least one selected from the group consisting of MNO₃, M₂SO₄, MCl and M₂CO₃, M being the alkali metal element;
preferably, the compound containing assistant metal element is at least one selected from the group consisting of oxide of the assistant metal and water-soluble salt containing the assistant metal.

15. The method according to any one of claims 9 to 14, wherein the mass percentage content of the pore-expanding agent is 3.9-4.2% based on the total amount of the second slurry;
preferably, the pore-expanding agent is at least one selected from the group consisting of stearic acid and sodium stearate.

16. The method according to any one of claims 9 to 15, wherein the coating temperature of the first slurry is higher than the coating temperature of the second slurry;
preferably, the coating temperature of the first slurry is T₁, the coating temperature of the second slurry is T₂, and T₁-T₂ is in the range of 70-150°C, preferably in the range of 80-120°C, more preferably in the range of 85-105°C;
preferably, the coating temperature of the first slurry is 200-250°C;
preferably, the coating temperature of the second slurry is 100-130°C;
preferably, a method for forming the second coating comprises purging the second slurry coated on the surface of the carrier where the first coating is formed with a purging gas stream, the purging gas stream preferably having a temperature of 380-450°C.

17. A catalyst prepared by the method according to any one of claims 9 to 16.

18. Application of the catalyst according to any one of claims 1 to 8 and 17 as a catalyst for a reaction for oxidation of 1,2,4,5-tetraalkylbenzene to prepare benzene-1,2,4,5-tetracarboxylic dianhydride.

19. A preparation method for benzene-1,2,4,5-tetracarboxylic dianhydride, comprising contacting 1,2,4,5-tetraalkylbenzene and an oxygen-containing gas with the catalyst according to any one of claims 1 to 8 and 17 to obtain a product gas stream containing benzene-1,2,4,5-tetracarboxylic dianhydride.
